# EUROPEAN PATENT APPLICATION

(11) **EP 3 950 662 A1**
(43) Date of publication of application: **09.02.2022**
(21) Application number: 20190089.1
(22) Date of filing: 07.08.2020
(51) Int. Cl.: C07C 227/18, C07C 229/12

(54) **METHOD FOR OBTAINING QUATERNARY AMMONIUM COMPOUNDS**

(71) Applicant: Rhodia Operations, 93300 Aubervilliers (FR)
(72) Inventor: BACK, Olivier, 69008 Lyon (FR)
(74) Representative: Ridray, Annabelle

(57) **Abstract**

The invention concerns a method for obtaining a quaternary ammonium compound. According to the invention, a diol of formula R^{h}-CH(OH)-CH(OH)-R^{h} wherein R^{h} represents an aliphatic group is reacted with an activated form of an amino-acid or derivative thereof or their hydrohalogenide adducts, such as an activated form of glycine or betaine hydrochloride.

## Description

The present invention relates to a new method for obtaining quaternary ammonium compounds. It relates also to new quaternary ammonium compounds which can be obtained by this method.

Fatty quaternary ammonium compounds which have surfactant properties and can be used in respective applications have been described in the literature and are available commercially in a variety of different types from various suppliers.

WO 97/08284 discloses compositions comprising Guerbet alcohol betaine esters which are represented by the general formula

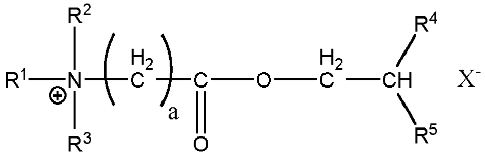

in which R¹ to R³ are independently selected from C₁ to C₄ alkyl groups or C₂-C₄ alkenyl groups, a is from 1 to 4 and R₄ and R₅ are independently selected from C₁₂ to C₂₂ alkyl or alkenyl groups, the sum of chain lenghts of R₄ and R₅ preferably being at least 30. Since the compounds are derived form Guerbet alcohols, the number of carbon atoms in groups R₄ and R₅ differs always by 2.

EP 721 936 and DE 3402146 relate to quaternary ammonium compounds. As in WO 97/08284 and EP 721 936, the compounds comprise two long chain substituents which are esters of Guerbet acids.

While fatty quaternary ammonium compounds are widely used as surfactants, there is still a need for compounds of this type having a good combination of surfactant properties on one hand and biodegradability on the other hand. Biodegradability has become more and more important in the recent past due to the desire of customers to have more environmentally friendly products. The improvement in biodegradability should not negatively affect the surfactant properties.

It was thus an object of the present invention to provide new quaternary ammonium compounds with good surfactant properties and a good biodegradability.

This object is achieved with the compounds of formula (I). Preferred embodiments of the present invention are also detailed hereinafter.

The novel ionic compounds in accordance with the present invention have the general formula (I) 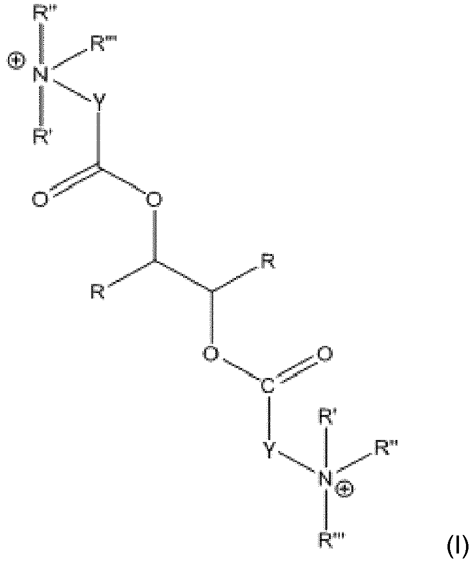 wherein
R, which may be the same or different at each occurrence, is a C₅-C₂₇ aliphatic group, preferably a C₆ to C₂₄ aliphatic group,
Y, which may be the same or different at each occurrence, is a divalent C₁-C₆ aliphatic radical,
R', R" and R'", which may be the same or different at each occurrence, are, independently from each other, hydrogen or a C₁ to C₄ alkyl group,

The aliphatic groups R may be free of any double bond and of any triple bond. Alternatively, the aliphatic groups R may comprise at least one -C=C- double bond and/or at least one -C=C- triple bond.

The aliphatic groups R are advantageously chosen from alkyl groups, alkenyl groups, alkanedienyl groups, alkanetrienyl groups and alkynyl groups.

The aliphatic groups R may be linear or branched.

Preferably, the aliphatic groups R are independently chosen from alkyl and alkenyl groups.

More preferably, the aliphatic groups R are independently chosen from alkyl and alkenyl groups, generally from C₆-C₂₄ alkyl and C₆-C₂₄ alkenyl groups, very often from C₆-C₂₁ alkyl and C₆-C₂₁ alkenyl groups and often from (i) C₆-C₁₉ alkyl and C₆-C₁₉ alkenyl groups or from (ii) C₆-C₁₇ alkyl and C₆-C₁₇ alkenyl groups. More preferably, R represent an alkyl group, generally a C₆-C₂₄ alkyl group, very often a C₆-C₂₁ alkyl group, often a C₆-C₁₉ alkyl group or a C₆-C₁₇ alkyl group. Aliphatic groups, in particular alkyl groups, with 10 to 20, preferably with 11 to 17 or with 10 to 17 carbon atoms have been found advantageous in certain cases.

Acyclic aliphatic groups, more preferably linear aliphatic groups, still more preferably linear alkyl groups may be mentioned as preferred examples of substituents R.

The number of carbon atoms of R can be even or odd and each group R can have the same number of carbon atoms or the number of carbon atoms of different groups R may be different.

R', R" and R'", which may be the same or different, are preferably hydrogen or a C₁ to C₄ alkyl group, preferably methyl or ethyl, more preferably methyl. Preferably at least one, more preferably at least two, more preferably all three of R', R" and R'" are a C₁ to C₄ alkyl group, preferably methyl or ethyl, most preferably methyl.

Y is preferably an acyclic divalent aliphatic group, more preferably a linear divalent aliphatic group, still more preferably a linear alkanediyl (alkylene) group and preferably has 1 to 6, even more preferably 1 to 4 carbon atoms. In compounds where n' is 1, aliphatic group Y preferably has at least two carbon atoms, in particular 2 to 6 carbon atoms.

The compounds of formula (II) 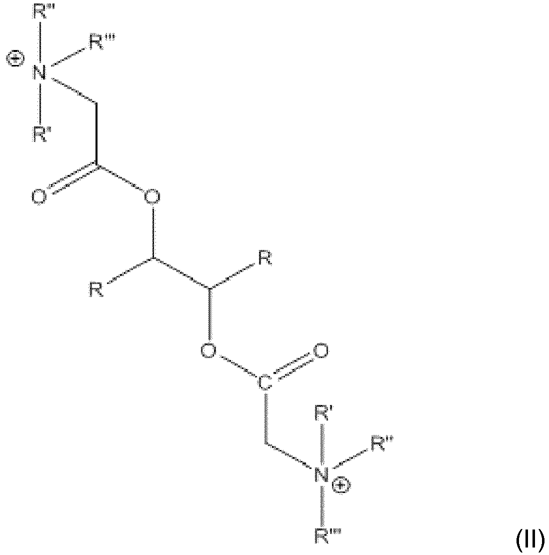 wherein R, R', R", R'" and Y in formula (II) have the meaning as defined for formula (I) as described hereinbefore represent particularly preferred compounds in accordance with the present invention.

The ionic compounds in accordance with the present invention can be obtained by a variety of methods. Preferred processes for the manufacture of the compounds of the present invention include the reaction of an internal ketone of formula R-C(=O)-R, which internal ketone may preferably be obtained by decarboxylative ketonization of a fatty acid, a fatty acid derivative or a mixture thereof. A suitable process for the manufacture of internal ketones following this route is diclosed in US 2018/0093936 to which reference is made for further details.

The synthesis of various compounds of the present invention using internal ketones obtainable as indicated above as starting materials is now described. The process variants described hereinafter show the synthesis of specific compounds and the skilled person will modify the reactants and reaction conditions based on his professional knowledge and taking into account the specific target product of the respective synthesis to manufacture other compounds in accordance with the present invention.

The ionic compounds of formula (I) can preferably be obtained by two processes. The first process starts with a *Piria ketonization* followed by hydrogenation, dehydration, epoxydation (to obtain an epoxide), hydration (to obtain a diol) and esterification (to obtain a certain diester). This is a multi-step process plugged on Piria technology. It has the advantage of being salt-free and relying on chemical transformations which can be easily performed.

As an alternative to the above sequence of reactions, it is possible to bypass the hydration step (i.e. the formation of the diol) by converting straightforward the epoxide into the diester provided an appropriate esterification agent is used, as will be detailed later on.

The esterification step may be followed by an amine condensation step (as the final step) to convert the diester into a compound complying with formula (I).

Finally, starting from the diol, it also possible to obtain in one reaction step a compound which complies with formula (I), i.e. to achieve in one step said esterification and amine condensation, provided another appropriate esterification is used, as will be detailed later on.

### First process for synthesis of compounds represented by formula (I)

### Piria Ketonization

The basic reaction in the first step is:

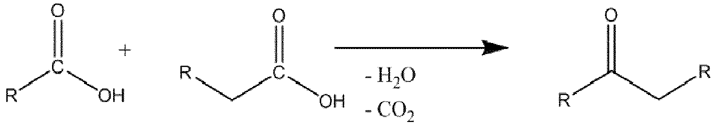

This reaction has been thoroughly described in US patent 10035746, WO 2018/087179 and WO 2018/033607 to which reference is made for further details.

### Hydrogenation

The internal ketone is then subjected to hydrogenation which can be carried out under standard conditions known to the skilled person for hydrogenation reactions:

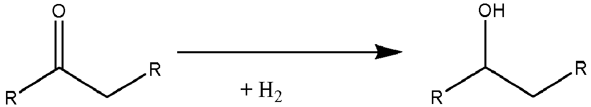

The hydrogenation reaction is conducted by contacting the internal ketone with hydrogen in an autoclave reactor at a temperature ranging from 15°C to 300°C and at a hydrogen pressure ranging from 1 bar to 100 bars. The reaction can be conducted in the presence of an optional solvent but the use of such solvent is not mandatory and the reaction can also be conducted without any added solvent. As examples of suitable solvents one can mention: methanol, ethanol, isopropanol, butanol, THF, methyl-THF, hydrocarbons, water or mixtures thereof. A suitable catalyst based on a transition metal should be employed for this reaction. As examples of suitable catalysts, one can mention heterogeneous transition metal based catalysts such as for example supported dispersed transition metal based catalysts or homogeneous organometallic complexes of transition metals. Examples of suitable transition metals are: Ni, Cu, Co, Fe, Pd, Rh, Ru, Pt, Ir. As examples of suitable catalysts one can mention Pd/C, Ru/C, Pd/Al₂O₃, Pt/C, Pt/Al₂O₃, Raney Nickel, Raney Cobalt etc. At the end of the reaction, the desired alcohol can be recovered after appropriate work-up. The skilled person is aware of representative techniques so no further details need to be given here. Details of this process step can e.g. be found in US patent 10035746 to which reference is made here.

The skilled person will select suitable reaction conditions based on his professional experience and taking into account the specific target compound to be synthesized. Accordingly, no further details need to be given here.

### Dehydration

In the next step, the alcohol thus obtained is subjected to dehydration to obtain an internal olefin. This reaction can also be carried out under standard conditions known to the skilled person for respective dehydration reactions (e.g. US patent 10035746, example 4) so that no further details need to be given here:

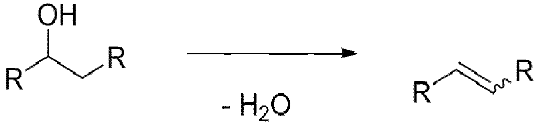

The dehydration reaction is conducted by heating the secondary alcohol in a reaction zone in the presence of a suitable catalyst at a temperature ranging between 100°C and 400°C. The reaction can be conducted in the presence of an optional solvent but the use of such solvent is not mandatory and the reaction can also be conducted without any added solvent. As examples of solvents one can mention: hydrocarbons, toluene, xylene or their mixture. A catalyst must be employed for this reaction. Suitable examples of catalysts are acidic (Lewis or Bronsted) catalysts either heterogeneous solid acid catalysts or homogeneous catalysts. As examples of heterogeneous catalysts one can mention alumina (Al₂O₃), silica (SiO₂), aluminosilicates (Al₂O₃-SiO₂) such as zeolites, phosphoric acid supported on silica or alumina, acidic resins such as Amberlite^{®} etc. Homogeneous catalysts can also be employed and one can mention the following suitable acids: H₂SO₄, HCI, trifluoromethanesulfonic acid, *para-*toluenesulfonic acid, AlCl₃, FeCl₃ etc. Water that is generated during the reaction can be distilled out from the reaction medium in the course of the reaction. At the end of the reaction, the desired olefin can be recovered after appropriate work-up. The skilled person is aware of representative techniques and same are e.g. described in US patent 10035746 so that no further details need to be given here.

### Epoxidation

This internal olefin can thereafter be oxidized to the respective epoxide wherein the double bond is substituted by an epoxide group in accordance with the following scheme (where the reactants are just exemplary for respective groups of compounds serving the respective function): 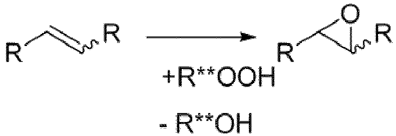 wherein R** can be hydrogen or a hydrocarbon group that can be substituted and/or interrupted by a heteroatom or heteroatom containing group, or R** can be an acyl group of general formula R***-C(=O)-wherein R*** can have the same meaning as R**.

The epoxidation reaction is advantageously conducted by contacting the internal olefin with an appropriate oxidizing agent in a reaction zone at a temperature ranging usually from 15°C to 250°C.

As suitable oxidizing agents one can mention peroxide compounds such as hydrogen peroxide (H₂O₂) that can be employed in the form of an aqueous solution, organic peroxides such as peracids of general formula R****-CO₃H (for example *meta*-chloroperoxybenzoic acid, peracetic acid, etc.), hydrocarbyl (e.g. alkyl) hydroperoxides of general formula R****'-O₂H (for example cyclohexyl hydroperoxide, cumene hydroperoxide, *tert*-butyl hydroperoxide) where R**** in the peracid or R****' in the hydrocarbyl (e.g. alkyl) hydroperoxide is a hydrocarbon group (e.g. an alkyl group) that can be substituted and/or interrupted by a heteroatom or heteroatoms-containing group.

The reaction can be conducted in the presence of an optional solvent but the use of such solvent is not mandatory and the reaction can also be conducted without any added solvent. As example of suitable solvents one can mention: CHCl₃, CH₂Cl₂, *tert*-butanol or their mixtures.

When H₂O₂ is used as the oxidizing agent, the presence of an organic carboxylic acid during the reaction can be beneficial as it will generate in-situ a peracid compound by reaction with H₂O₂. As examples of suitable carboxylic acids one can mention: formic acid, acetic acid, propionic acid, butanoic acid, benzoic acid etc.

A catalyst can also be used to promote the reaction. Suitable catalysts are Lewis or Bronsted acids and one can mention for example: perchloric acid (HClO₄), trifluoromethanesulfonic acid, heterogeneous titanium silicalite (TiO₂-SiO₂), heterogeneous acidic resins such as Amberlite^{®} resins, homogeneous organometallic complexes of manganese, titanium, vanadium, rhenium, tungsten, polyoxometellates etc.

At the end of the reaction, the desired epoxide can be recovered after appropriate work-up and the skilled person is aware of representative techniques so that no further details need to be given here.

The epoxide can be directly engaged in next step without further purification. This next step can be a hydration step (so as to form a diol, to be the followed by an esterification step) or a direct esterification step.

### Epoxide hydration (diol formation)

The epoxide can thereafter be hydrated to the respective diol in accordance with the following scheme:

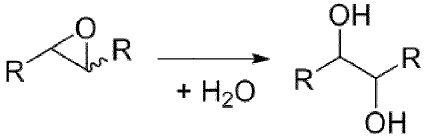

The ring opening reaction can be performed by contacting the epoxide with water, generally in the presence of a suitable catalyst and at a temperature ranging generally from 15°C to 150°C. As examples of catalysts one can mention Bronsted or Lewis acid catalysts such as: H₂SO₄, HCI, perchloric acid (HClO₄), trifluoromethanesulfonic acid, *para-*toluenesulfonic acid, heterogeneous acidic resins such as Amberlite^{®} resins etc.

The reaction can be conducted in the presence of an optional solvent to facilitate reagent contact and one can mention: Me-THF, THF, DMSO, *tert-*butanol, methanol, ethanol, isopropanol, acetonitrile, or their mixture. The reaction can also be conducted without any added solvent.

At the end of the reaction, the desired diol can be recovered after appropriate work-up and the skilled person is aware of representative techniques so that no further details need to be given here.

### Esterification (starting from the diol)

The diol can be esterified according to the following reaction scheme: 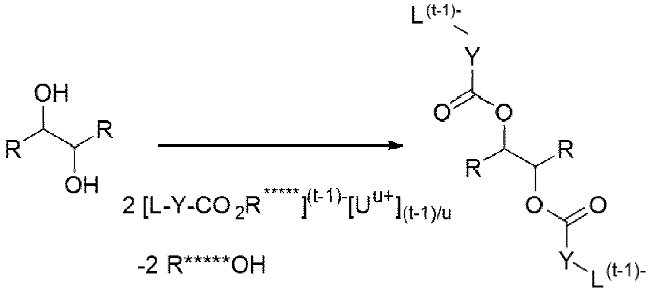 wherein
R, which may be the same or different at each occurrence, is a C₅-C₂₇ aliphatic group, preferably a C₆ to C₂₄ aliphatic group,
L is a leaving group,
Y is a divalent C₁-C₆ aliphatic radical,
R***** is hydrogen or a C₁-C₆ alkyl group,
t is an integer which is equal to 1 or which is equal or superior to 2,
U^{u+} is a cation, and
u is an integer fixing the positive charge of the cation.

The esterification is first performed by contacting the diol with an esterification agent which is a carboxylic acid or an ester of a carboxylic acid of general formula:

[L-Y-CO₂R*****]^{(t-1)}-[U^{u+}]_{(t-1)/u}

wherein Y is a divalent hydrocarbon radical containing between 1 and 6 carbon atoms, more precisely a divalent C₁-C₆ aliphatic radical, and wherein L is a leaving group.

Y is preferably an acyclic divalent aliphatic group, more preferably a linear divalent aliphatic group, still more preferably a linear alkanediyl (alkylene) group. Y has preferably from 1 to 6, more preferably from 1 to 4 carbon atom(s), still more preferably 1 or 2 carbon atom(s). The most preferred Y is -CH₂-.

When t is equal to 1, no cation is present. Otherwise said, the esterification is performed by contacting the diol with a carboxylic acid or an ester of a carboxylic acid of formula:

L-Y-CO₂R*****.

In the case the leaving group L already carries a negative charge in the carboxylic acid or ester reactant (this is the case when (t-1) is equal or superior to 1 or when t is equal or superior to 2), a cation noted U^{u+} (with u preferably being 1, 2 or 3, even more preferably 1) must be present in the reactant to ensure the electroneutrality (in this case the cation possesses a u⁺ charge). This cation may e.g. be selected from H⁺, alkaline metal cations, alkaline earth metal cations (e.g. Na⁺, K⁺, Ca ²⁺), Al³⁺ and ammonium, to mention only a few examples.

The nature of the leaving group L is not particularly limited provided next reaction step (i.e. amine condensation, as will be detailed later on) can occur. The leaving group L is preferably chosen from:
- a halogen, such as fluorine, chlorine, bromine or iodine,
- a (hydrocarbyloxysulfonyl)oxy group of formula R^{a}-O-SO₂-O- wherein R^{a} denotes a C₁-C₂₀ hydrocarbyl group, such as CH₃-O-SO₂-O-, and
- an oxysulfonyloxy group of formula -O-SO₂-O-.

An example for a compound with t equal to 1 is the compound CH₃-O-SO₃-CH₂-COOR***** which, for R***** being H, yields the compound CH₃-O-SO₃-CH₂-COOH which can be designated as 2-((methoxysulfonyl)oxy)acetic acid.

As further examples of compounds in which t is equal to 1 and thus no cation is present, one can mention: chloroacetic acid, bromoacetic acid and 2-chloropropionic acid.

An example for t being equal to 2 is sodium carboxymethylsulfate acid in which [L-Y-COOR*****]^{(t-1)-}[U^{u+}]_{(t-1)/u} is [Na⁺] [O-SO₂-O-CH₂-COOR*****]⁻ with R***** being H, U being Na and thus [U^{u+}]_{t/u[}L^{t-}] being Na₂SO₄.

The esterification can be conducted at a temperature ranging from 50°C to 250°C in the presence of an optional solvent. As example of suitable solvents one can mention toluene, xylene, hydrocarbons, DMSO, etc.

Water that is formed as a by-product during the reaction can be removed from the reaction medium by distillation over the course of the reaction.

A catalyst can also be employed during the reaction and suitable catalysts are Bronsted or Lewis acid catalysts. As preferred examples of catalysts one can mention: H₂SO₄, *para*-toluenesulfonic acid, trifluoromethanesulfonic acid, HCI, or heterogeneous acidic resins such as Amberlite^{®} resins, AlCl₃ etc. At the end of the reaction, the desired diester can be recovered after appropriate work-up and the skilled person is aware of representative techniques so that no further details need to be given here.

As will be seen later on, the diester of formula (III) 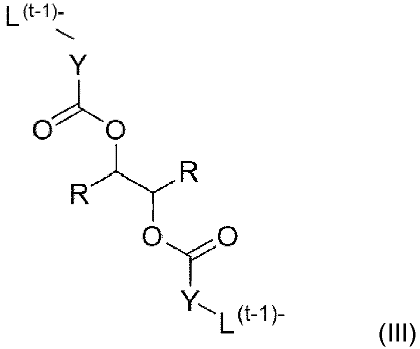 can be easily converted into a compound of formula (I) as previously represented, which exhibits outstanding surfactant properties.

### Direct esterification (starting from the epoxide, including the epoxide ring-opening)

The Applicant has surprisingly found that the epoxide can be directly esterified into the diester of formula (III) when and only when a carboxylic acid is used as the esterification agent [L-Y-CO₂R*****]^{(t-1)}-[U^{u+}]_{(t-1)/u}, that is to say when the esterification agent [L-Y-CO₂R*****]^{(t-1)}-[U^{u+}]_{(t-1)/u} is of the formula
[L-Y-CO₂H]^{(t-1)}-[U^{u+}]_{(t-1)/u}, wherein L, Y, t, U^{u+} and u are as described here before in connection with the esterification of the diol.

The epoxide ring-opening and esterification take then place according to the following reaction scheme: 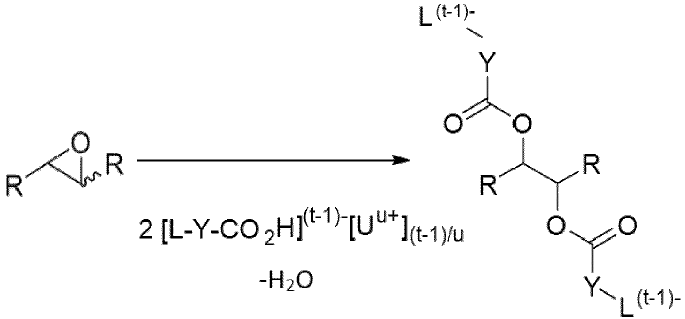 wherein
R, which may be the same or different at each occurrence, is a C₅-C₂₇ aliphatic group, preferably a C₆ to C₂₄ aliphatic group,
L is a leaving group,
Y is a divalent C₁-C₆ aliphatic radical,
t is an integer which is equal to 1 or which is equal or superior to 2,
U^{u+} is a cation, and
u is an integer fixing the positive charge of the cation.

The epoxide ring-opening and esterification are performed by reacting the epoxide with a carboxylic acid of general formula:

[L-Y-CO₂H]^{(t-1)-}[U^{u+}]_{(t-1)/u}

wherein L is a leaving group, Y is a divalent C₁-C₆ aliphatic radical, t is an integer which is equal to 1 or which is equal or superior to 2, U^{u+} is a cation, and u is an integer fixing the positive charge of the cation.

All the details which have been provided here before concerning L, Y, t, U^{u+} and U in connection with the esterification of the diol are also valid and can apply here in connection with the direct esterification of the epoxide, so that such details need not to be repeated.

In particular, when t is equal to 1, no cation is present. Otherwise said, the esterification is performed by contacting the epoxide with a carboxylic acid of formula:

L-Y-CO₂H.

### Amine condensation from the diester

The diester compound of formula (III) can be converted into the ionic compound of formula (I) through the following reaction scheme: 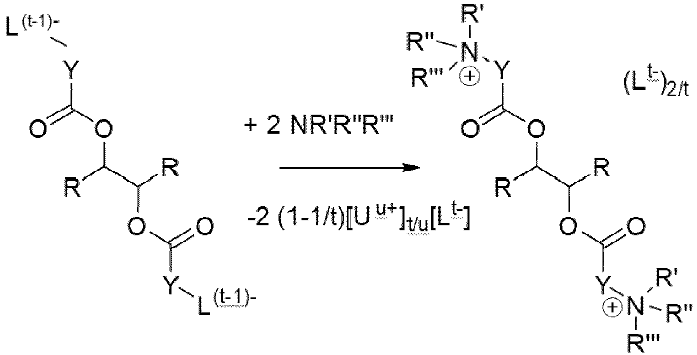 wherein
- R, Y, L, t, U^{u+} and U are as described here before, and
- R', R" and R'" are C₁ to C₄ alkyl groups, preferably methyl or ethyl, most preferably methyl.

The amine condensation reaction is performed by contacting the intermediate diester of formula (III) obtained as described above with an amine of general formula NR'R"R'" where R', R" and R'" are C₁ to C₄ alkyl groups, preferably methyl or ethyl, most preferably methyl.

The reaction can be conducted at a temperature ranging from 15°C to 250°C in the presence of a suitable solvent. As example of a suitable solvent one can mention: THF, Me-THF, methanol, ethanol, isopropanol, DMSO, toluene, xylene or their mixture. Alternatively the reaction can be also conducted in the absence of any added solvent.

During this reaction, there is a nucleophilic attack of the amine that substitues L^{(t-1)-} in the diester, L^{(t-1)-} plays the role of the leaving group. L^{t-} becomes then the counter-anion of the final quaternary ammonium compound. In the case the leaving group already carries a negative charge in the diester reactant (this is the case when (t-1) is equal or superior to 1 or when t is equal or superior to 2) there is also formation of a salt as the by-product of the reaction (with the general chemical formula [U^{u+}]_{t/u}[L^{t-}] as shown in the equation scheme above).

### Esterification and amine condensation / direct quaternization from the diol

Starting from the diol of formula 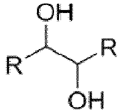 wherein R is as described here before, the Applicant has surprisingly found that it is possible to obtain in one reaction step a compound which complies with formula (I), i.e. to achieve in one step said esterification and amine condensation, provided another appropriate esterification agent is used.
This appropriate esterification agent can be generally defined as an activated form of an amino-acid or an ester thereof of formula (IVa)

R'R"R'" ⁺N-Y-C(=O)-O⁻ (IVa)

or their hydrohalogenide adducts of formula (IVb)

R'R"R'" ⁺N-Y-C(=O)-OH X⁻ (IVb)

wherein
- R', R" and R'", which may be the same or different, are hydrogen or a C₁ to C₄ alkyl group,
- Y is a divalent C₁-C₆ aliphatic radical, and
- X denotes a halogen atom, such as chlorine or bromine.

The activated form of the amino-acid or ester thereof of formula (IVa) or their hydrohalogenide adducts of formula (IVb) is advantageously an acyl halogenide of formula (V)

R'R"R'" ⁺N-Y-C(=O)-X X⁻ (V)

wherein R', R", R'", Y and X are as described before in connection with formulae (IVa) and (IVb) ; in particular, it can be an acyl chloride of formula R'R"R'" ⁺N-Y-C(=O)-Cl Cl⁻.

Other suitable activated forms of the the amino-acid or ester thereof of formula (IVa) or their hydrohalogenide adducts of formula (IVb) include an acyl azide of formula (VI)

R'R"R'" ⁺N-Y-C(=O)-N=N⁺=N⁻ (VI)

and an acyl imidazole or acyl imidazolium.

Several publications describe the direct esterification of a polyol wherein the hydroxyl groups are *primary* (emphasis added) hydroxyl groups with an amino-acid or an ester thereof.

For example, Stakleff in Acta Biomaterialia, 9, 5132-5142 (2013), taught about the reaction of hexanediol with phenylalanine or leucine in the presence of p-toluene sulfonic acid.

Lele et al., in Synthetic Communications, 29:10, 1727-1739 (1999) achieved the mediated coupling of poly(ethylene glycol) and an amino-acid hydrochloride in the presence of dicyclohexylcarbodiimide.

Granö et al., in Carbohydrate Polymers, 41, 277-283 (2000), prepared starch betainate from starch and betainyl chloride in the presence of dioxane as solvent and pyridine as nucleophilic reagent and catalyst: 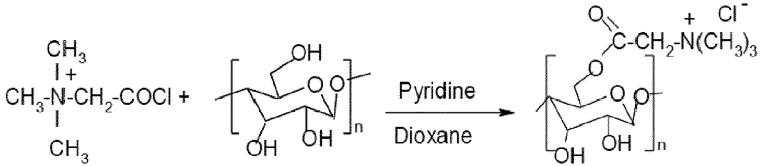 Betainyl chloride had been earlier prepared by reacting glycine betaine with thionyl chloride in dichloromethane.

On the other hand, none of these pieces of prior art addresses the possibility to quaternize directly a polyol bearing two or more secondary hydroxyl groups, the reactivity of which differs substantially from the reactivity of polyols bearing primary hydroxyl groups, as it could now be achieved within the frame of the present invention.

It was thus another object of the present invention to provide a new, efficient and easy method for the direct quaternization of diols of formula 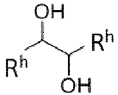 wherein R^{h}, which may be the same or different at each occurrence, represent an aliphatic group. In particular, it was another object of the present invention to a provide a new, efficient and easy method for the direct quaternization of diols of formula 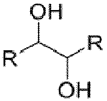 (with R as described herein before) whereby quaternary ammonium compounds with good surfactant properties and a good biodegradability are obtained.

This other object is achieved with a method which is described hereinafter under item 1:
- item 1 : a method for obtaining a quaternary ammonium compound having the general formula 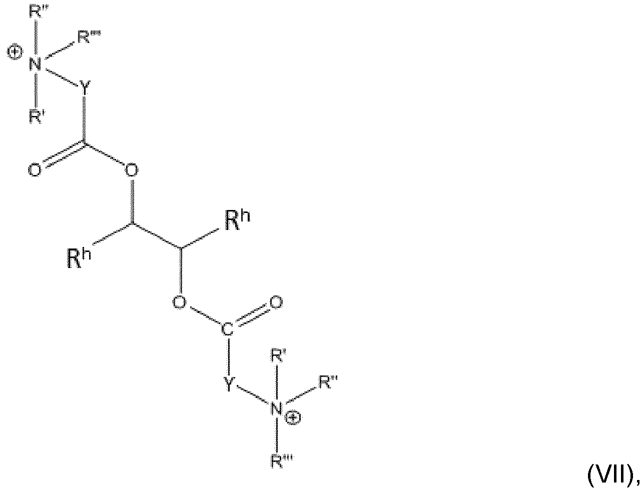 said method comprising reacting a diol of formula 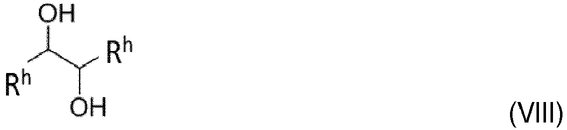 with an activated form of an amino-acid or derivative thereof or their hydrohalogenide adducts,
   wherein
   - the amino-acid or derivative thereof is of formula (IVa)

      R'R"R'" ⁺N-Y-C(=O)-O⁻ (IVa),

      and their hydrohalogenide adducts are of formula (IVb)

      R'R"R'" ⁺N-Y-C(=O)-OH X⁻ (IVb),
   - R^{h}, which may be the same or different at each occurrence, is an aliphatic group
   - R', R" and R'" are hydrogen or a C₁ to C₄ alkyl group,
   - Y is a divalent C₁-C₆ aliphatic radical, and
   - X denotes a halogen atom, such as chlorine or bromine.
   It is understood that in formula (VII), as it is the case for R^{h}, Y, R', R" and R'" may be the same or different at each occurrence.
   Preferred and/or particular embodiments related to the invented method are set hereinafter:
- item 2: the method as described in item 1 wherein the activated form of the amino-acid or derivative thereof or their hydrohalogenide adducts is an acyl halogenide of formula (V)

   R'R"R'" ⁺N-Y-C(=O)-X X⁻ (V)

   wherein R', R", R'", Y and X are as described in item 1;
- item 3: the method as described in item 2 which comprises obtaining the acyl halogenide of formula (V) by reacting the amino-acid or derivative thereof of formula (IVa) and/or a hydrohalogenide adduct thereof of formula (IVb) with a reagent chosen from a thionyl halogenide, an oxalyl halogenide, a phosphorus trihalogenide, a phosphorus pentahalogenide or a phosphorus oxyhalogenide, preferably by reacting the amino-acid or derivative thereof of formula (IVa) and/or a hydrohalogenide adduct thereof of formula (IVb) with a reagent chosen from thionyl chloride (SOCl₂), oxalyl chloride [(COCl)₂], phosphorus trichloride (PCl₃), phosphorus pentachloride (PCl₅) and phosphorus oxychloride (POCl₃), more preferably by reacting the amino-acid or derivative thereof of formula (IVa) and/or a hydrohalogenide adduct of formula (IVb) with thionyl chloride ;
- item 4: the method as described in item 1 wherein the activated form of the amino-acid or derivative thereof or their hydrohalogenide adducts is an acyl azide of formula (VI)

   R'R"R'" ⁺N-Y-C(=O)-N=N⁺=N⁻ (VI)

   wherein R', R", R'" and Y are as described before in item 1 ;
- item 5: the method as described in any one of items 1 to 4 wherein X is chloride ;
- item 6: the method as described in any one of items 1 to 5 wherein R^{h} comprises at least 2, possibly at least 3 or at least 4 carbon atoms ;
- item 7: the method as described in any one of items 1 to 6 wherein R^{h} comprises up to 4 carbon atoms ;
- item 8: the method as described in any one of items 1 to 6 wherein R^{h} comprises at least 5 carbon atoms ;
- item 9: the method as described in item 8 wherein R^{h} (i.e. each R^{h}), which may be the same or different at each occurrence, represents a C₅-C₂₇ aliphatic group, i.e. R^{h} has the same definition as R as previously described, and the quaternary ammonium compound which is obtained by the present method is of formula (I) ;
- item 10: the method as described in item 9 wherein R^{h} complies with any one of the features which are specified hereafter in sub-items 10.a to 10.g or with any combination of two, three or more of such features, to the extent that so-combined features are not antagonistic with each other:
   - sub-item 10.a: R^{h} (i.e. each R^{h}) is a C₆ to C₂₄ aliphatic group, preferably R^{h} comprises from 10 to 20 carbon atoms, more preferably R^{h} comprises from 11 to 17 carbon atoms ;
   - sub-item 10.b: R^{h} is free of any double bond and of any triple bond, preferably R^{h} is an alkyl group ;
   - sub-item 10.c: R^{h} is linear, preferably R^{h} is a linear alkyl group having from 14 to 17 carbon atoms ;
   - sub-item 10.d: one and only one R^{h} has an odd number of carbon atoms and one and only one R^{h} has an even number of carbon atoms ; this can happen when both R^{h} originate from a carboxylic acid having an even number of carbon atoms and can be advantageous from an economic standpoint because fatty carboxylic acids of natural origin -which have typically such an even number of carbon atoms- are broadly available ; this can also happen when both R^{h} originate from a carboxylic acid having an odd number of carbon atoms ;
   - sub-item 10.e: either both R^{h} have an even number of carbon atoms or both R^{h} have an odd number of carbon atoms ; this can happen when one and only one R^{h} originates from a carboxylic acid having an even number of carbon atoms and one and only one R^{h} originates from a carboxylic acid having an odd number of carbon atoms ;
   - sub-item 10.f: one and only one R^{h} has an odd number of carbon atoms no while the other R^{h} has an even number of carbon atoms n_{E}, wherein n_{E} is equal to no-1 ; this can happen when R^{h} originates from one and only one carboxylic acid ;
   - sub-item 10.g: the number of carbon atoms of the two R^{h} groups is represented by couple (n₁, n₂), n₁ being the number of carbon atoms of the first R^{h} group and n₂ being the number of carbon atoms of the second R^{h} group, said couple (n₁, n₂) being chosen from the following couples: (10,11), (12,13), (14,15), (16,17), (10,13), (10,15), (10,17), (11,12), (11,14), (11,16), (12,15), (12,17), (13,14), (13,16), (14,17) and (15,16) ; this can happen when R^{h} originates from a cut of C₁₂-C₁₈ fatty carboxylic acids of natural origin ;
- item 11: the method as described in any one of items 1 to 10 wherein Y is chosen -CH₂-, -CH(CH₃)-, -CH[CH(CH₃)₂]-, -CH[CH₂-CH(CH₃)₂]- and -CH[CH(CH₃)-CH₂-CH₃]- ;
- item 12: the method as described in item 11 wherein Y is -CH₂- ;
- item 13: the method as described in any one of items 1 to 12 wherein at least one of R', R" and R'" is a C₁ to C₄ alkyl group ;
- item 14: the method as described in item 13 wherein R' is a C₁ to C₄ alkyl group, R" is a C₁ to C₄ alkyl group and R'" is a C₁ to C₄ alkyl group ;
- item 15: the method as described in item 14 wherein R', R" and R'" are methyl ;
- item 16: the method as described in any one of items 1 to 15 wherein the amino-acid or derivative thereof of formula (IVa) is trimethyl glycine and the hydrohalogenide adduct of formula (IVb) is betaine hydrochloride.

### Second process for synthesis of compounds represented by formula (I)

### Acyloin condensation

An alternative process for the synthesis of compounds of formula (I) proceeds via an acyloin condensation in accordance with the following scheme: 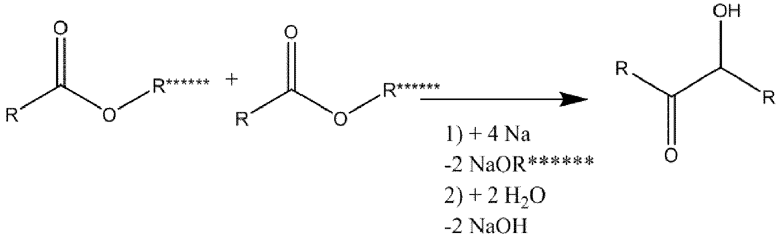 wherein R****** is an alkyl group having from 1 to 6 carbon atoms.

### Keto-alcohol hydrogenation

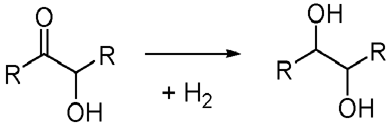

This reaction can be conducted using the conditions described hereinbefore for the first process variant for the manufacture of compounds of formula (I).

The subsequent reaction steps are also as described hereinbefore for the first process variant for the manufacture of compounds of formula (I).

The exemplary processes described before are examples of suitable processes, i.e. there might be other suitable processes to synthesize the compounds in accordance with the present invention. The processes described hereinbefore are thus not limiting as far as the methods of manufacture of the compounds according to the present invention is concerned.

In the compounds of formula (I), the number of carbon atoms of the two groups R is preferably any of the following couples if the internal ketones used as reactant in the exemplary processes described hereinbefore are derived from natural fatty acids having an even number of carbon atoms:
(4,5), (6,7), (8,9), (10,11), (12,13), (14,15), (16,17)
(4,7), (4,9), (4,11), (4,13), (4,15), (4,17)
(5,6), (5,8), (5,10), (5,12), (5,14), (5,16)
(6,9), (6,11), (6,13), (6,15), (6,17)
(7,8), (7,10), (7,12), (7,14), (7,16)
(8,11), (8,13), (8,15), (8,17)
(9,10), (9,12), (9,14), (9,16)
(10,13), (10,15), (10,17)
(11,12), (11,14), (11,16)
(12,15), (12,17)
(13,14), (13,16)
(14,17)
(15,16).

In particular, if the internal ketones used as reactant in the exemplary processes described hereinbefore are obtained from one or more carboxylic acids having an even number of carbon atoms ranging from 12 to 18, the number of carbon atoms of the two groups R in compounds of formula (I) is one or more of the following couples:
(10,11), (12,13), (14,15), (16,17)
(10,13), (10,15), (10,17)
(11,12), (11,14), (11,16)
(12,15), (12,17)
(13,14), (13,16)
(14,17)
(15,16)

If the internal ketone is obtained from fatty acids comprising an odd numbers of carbon atoms, other couples are possible and will be obtained.

Compounds of formula (I) possess a particularly interesting and advantageous property profile of surfactant properties on one hand and biodegradability properties on the other hand. Biodegradability is becoming more and more an important aspect for surfactant products.

The compounds of the present invention can be used as surfactants. Surfactants are compounds that lower the surface tension (or interfacial tension) between two liquids, a liquid and a gas or between a liquid and a solid. Surfactants may act as detergents, wetting agents, emulsifiers, foaming agents, and dispersants.

Surfactants are usually organic compounds that are amphiphilic, meaning they contain both hydrophobic groups (their *tails*) and hydrophilic groups (their *heads*). Therefore, a surfactant contains both a water-insoluble (or oil-soluble) component and a water-soluble component. Surfactants will diffuse in water and adsorb at interfaces between air and water or at the interface between oil and water, in the case where water is mixed with oil. The water-insoluble hydrophobic group may extend out of the bulk water phase, into the air or into the oil phase, while the water-soluble head group remains in the water phase.

The adsorption of a cationic surfactant on negatively charged surfaces is an important property for such surfactants. This property is usually linked to the minimum concentration of surfactant needed to produce aggregation of a negatively charged cellulose nanocrystal (CNC, which is often used as reference material)) suspension in aqueous media. Consecutive variation of size can be monitored and followed by dynamic light scattering (DLS).

Following the protocol described in E.K. Oikonomou et al., J. Phys. Chem. B, 2017, 121 (10), 2299-307 the adsorption properties of the quaternary ammonium compounds can be investigated by monitoring the ratio X=[surfactant]/[CNC] or the mass fraction M=[surfactant]/([surfactant + [CNC]), at fixed [surfactant] + [CNC] = 0.01 wt% in aqueous solution, required to induce the agglomeration of the cellulose nanocrystals.

The biodegradability of the compounds of the present invention can be determined in accordance with procedures described in the prior art and known to the skilled person. Details about one such method, OECD standard 301, are given in the experimental section hereinafter.

Should the disclosure of any patents, patent applications, and publications which are incorporated herein by reference conflict with the description of the present application to the extent that it may render a term unclear, the present description shall take precedence.

### Working examples

### Example 1 - Synthesis of a quaternary ammonium compound corresponding to formula (I) starting from C₃₁ 16-hentriacontanone

C₃₁ internal olefin was obtained from palmitic acid according to the protocol described in US patent 10035746, example 4.

### Epoxidation of internal olefin to fatty epoxide

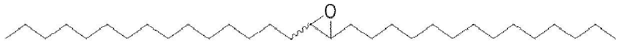

The reaction was conducted under an inert argon atmosphere.

In a 1L double-jacketed reactor equipped with a mechanical stirrer (propeller with four inclined plows), a condenser, an addition funnel and a temperature probe were added 61.9 g of C₃₁ alkene (0.142 mol), followed by 16.3 mL (17.1 g, 0.285 mol) of acetic acid and 13.6 g (22 wt%) of Amberlite^{®} IR 120H resin. The mixture was heated to 65 °C to melt the fatty alkene. The agitation was started and then 21.8 mL (24.2 g, 0.214 mol) of an aqueous solution of H₂O₂ (conc. 30%) was slowly added to the mixture using the addition funnel at a rate avoiding a significant temperature increase. This required about one hour. The temperature was then increased to 75°C and the reaction mixture was allowed to stir overnight (after 15 min, NMR analysis showed that the conversion level was already around 60% with 99% selectivity). Then additional 10.2 mL (11.3 g, 0.1 mol) of an aqueous solution of H₂O₂ (30%) was added slowly and after 4 hours following the second addition of H₂O₂ NMR analysis showed that the conversion level was around 88% (98% selectivity). Another addition of 8.14 mL of acetic acid (8.55 g, 0.142 mol) followed by 11.6 mL of 30% H₂O₂ (12.91 g, 0.114 mol) was finally performed in order to increase the conversion level.

The mixture was allowed to stir a second night at 75°C.

Finally NMR analysis showed a conversion level of 93% (95% selectivity).

The mixture was allowed to cool down to room temperature and then 300 mL of chloroform were added. The mixture was transferred to a separating funnel and the organic phase was washed three times with 300 mL of water and then the aqueous phase was extracted twice with 100 mL of chloroform. The Amberlite^{®} solid catalyst stayed in the aqueous phase and was removed during the first separation with the aqueous phase. The organic phases were collected, dried over MgSO₄, filtered and evaporated to give 65.3 g of a white solid with a purity of 91% w/w (epoxide + di-alcohol).

The yield taking into account the purity was 92%.
¹H NMR (CDCl₃, 400 MHz) δ (ppm): 2.91-2.85 (m, 2H, diastereoisomer 1), 2.65-2.6 (m, 2H, diastereoisomer 2), 1.53-1.00 (m, 54H), 0.86 (t, J = 6.8 Hz, 6H).
¹³C NMR (CDCl₃, 101 MHz) δ (ppm): 58.97, 57.28, 32.18, 31.96, 29.72, 29.6, 29.4, 27.86, 26.95, 26.63, 26.09, 22.72, 14.15 (terminal CH₃).

### Hydrolysis of fatty epoxide to afford fatty diol

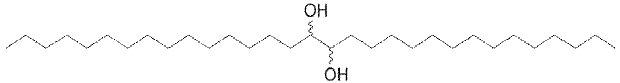

The reaction was conducted under an inert argon atmosphere.

In a 1L double-jacketed reactor equipped with a mechanical stirrer (propeller with four inclined plows), a condenser and a temperature probe were added 82.9 g of C₃₁ epoxide (purity: 94.5 wt%, 0.174 mol) followed by 480 mL of methyl-THF.

The mixture was allowed to stir at room temperature and 73 mL of a 3 M aqueous solution of H₂SO₄ was then added. The reaction medium was then stirred at 80°C during 90 minutes. NMR analysis showed that the reaction was completed. The biphasic mixture was allowed to cool down to room temperature and the organic phase was separated. The solvent was then removed under vacuum and the residue was suspended in 200 mL of diethyl ether. The suspension was filtered and the resulting solid was washed 3 times with 50 mL of diethyl ether. The white solid was finally washed 2 times with 50 mL of methanol and was dried under vacuum to remove traces of solvent.

At the end 75.53 g of product was obtained as a white powder with a purity of 95.7 % w/w corresponding to a yield of 89 %.
¹H NMR (CDCl₃, 400 MHz) δ (ppm): 3.61-3.55 (m, 2H, diastereoisomer 1), 3.43-3.25 (m, 2H, diastereoisomer 2), 1.88 (brd, *J* = 2.4 Hz, OH, diastereoisomer 2), 1.72 (brd, *J* = 3.2 Hz, OH, diastereoisomer 1), 1.53-1.10 (m, 54H), 0.86 (t, *J* = 6.8 Hz , 6H).
¹³C NMR (CDCl₃, 101 MHz) δ (ppm): 74.71, 74.57, 33.66, 31.96, 31.23, 29.71, 29.39, 26.04, 25.68, 22.72, 14.15 (terminal CH₃)

### Esterification and quaternization of fatty diol with trimethylglycine to afford compound of formula (I)

All the reactions were conducted in carefully dried vessels and under an inert argon atmosphere.

Fresh commercial anhydrous CHCl₃ (amylene stabilized) and anhydrous toluene were used as such.

Betaine hydrochloride (19.66 g, 128.4 mmoles) was washed ten times with 20 mL of anhydrous THF followed by drying under vacuum to remove traces of solvent prior to use.

In a 100 mL four-neck round-bottom flask equipped with a magnetic stirrer, a heater, a condenser, a temperature probe and a curved distillation column connected to two traps of NaOH were quickly added:
19.66 g of dried betaine hydrochloride (128.4 mmoles) and
28 mL of SOCl₂ (45.86 g, 0.386 mol).

The heterogeneous mixture was stirred and the temperature was then slowly increased to 70°C. It was observed that when the temperature reached 68°C, gas was released (SO₂ and HCI) and the mixture turned homogeneous yellow.

The mixture was then allowed to stir at 70°C during two hours and hot anhydrous toluene (25 mL, 80°C) was added into the vessel. The mixture was stirred and then decanted at 0°C (white-yellow precipitate formation) and the upper phase of toluene was removed through a cannula. The operation of toluene washing was repeated seven times in order to remove all SOCl₂ excess. NMR analysis showed complete conversion of glycine betaine hydrochloride but also formation of NMe₃·HCl adduct (NMe₃·HCl content in the solid: 12.3 mol%).

20 mL of dry CHCl₃ was then added to the solid betainyl chloride.

A solution of 26.19 g (56 mmol) of fatty diol in 90 mL of anhydrous CHCl₃ was prepared at 55°C and was added dropwise under stirring to the reaction vessel at room temperature (exothermicity and emission of HCI was observed). The mixture was then allowed to stir at 55°C overnight. Over the course of the reaction, the mixture turned homogeneously orange. NMR analysis showed that the conversion level was around 100%.

The mixture was then allowed to cool down to room temperature and the solvent was evaporated under vacuum.

The residue was solubilized in methanol at 0°C and the formed precipitate was filtered out. The obtained filtrate was then evaporated to give 39.7 g of crude product.

This product was then deposited on a sinter filter and washed with cyclohexane to remove some remaining organic impurities. The resulting washed solid was dried under vacuum to afford 22 g of crude material. A final purification with a mixture of CH₂Cl₂/cyclohexane 50:50 was carried out; the solid was solubilized again in this solvent mixture at 50°C and was allowed to cool down to room temperature. The formed precipitate was filtered out and after evaporation of the filtrate 19 g of a beige wax was obtained with the following composition:
95 wt% of glycine betaine diester
1.5 wt% of methyl betainate
2 wt% of trimethylamine hydrochloride
1.5 wt% of glycine betaine hydrochloride.

The purified yield was 44%.
¹H NMR (MeOD-d4, 400 MHz) δ (ppm): 5.3-5.2 (m, 2H), 4.68 (d, J = 16.8 Hz, 2H), 4.50 (d, J = 16.8 Hz, 2H), 4.53 (s, 1H), 4.48 (s, 1H), 3.37 (s, 18H), 1.75-1.55 (m, 4H), 1.39-1.10 (m, 50 H), 0.9 (t, J = 6.8 Hz, 6H).
¹³C NMR (MeOD-d4, 101 MHz) δ (ppm): 164.58, 75.76, 62.43, 53.10, 31.68, 30.05, 29.41, 29.38, 29.33, 29.28, 29.15, 29.09, 28.96, 24.71, 22.34, 13.05 (terminal CH₃).

### Example 2 - Evaluation of adsorption properties on Nanocellulose crystals

Adsorption of cationic surfactant on negatively charged surface is an important property for various applications. This property is linked to the minimal concentration of cationic surfactant needed to produce aggregation of negatively charged cellulose nano crystal (CNC) in suspension in aqueous media. Comparison of the aggregate size can be monitored by dynamic light scattering (DLS).

Following the protocol described in literature (Ref.: E. K. Oikonomou, et al., J. Phys. Chem. B, 2017, 121 (10), pp 2299-2307), adsorption properties of quaternary ammonium were investigated by monitoring the ratio X=[surfactant]/[CNC] or the mass fraction M = [surfactant]/([surfactant]+[CNC]), at fixed [surfactant]+[CNC]= 0.01wt% in aqueous solution, required to induce the agglomeration of the cellulose nano crystal.

The range of CNC aggregation correspond to the range of ratio X (or M) triggering an aggregation of CNC, i.e. the range where the aggregate size measured by DLS is higher than a pure aqueous solution of CNC or an aqueous solution of surfactant at 0.01wt%.

Ranges of X and M of aggregation of CNC are summarized in Table 1. The lower range of aggregation X or M, the better the adsorption properties on negatively charged surface

**Table 1**

| | Range of CNC aggregation (Ratio) X=[surfactant]/[CNC] Xₘᵢₙ - Xₘₐₓ | Range of CNC aggregation (Mass fraction) Mmin - Mmax |
|---|---|---|
| Compound of Example 1 | 0.1-1.82 | 0.09-0.65 |
| Fentacare^{®} TEP | 1-33 | 0.50-0.97 |

Fentacare^{®} TEP was used as a comparison. Fentacare^{®} TEP is a commercial surfactant representing the benchmark.

The data show that the surfactant properties of the compound in accordance with the present invention is superior compared to the commercial surfactant Fentacare^{®} TEP.

### Example 3 - Determination of biodegradability

Biodegradability of the test substances has been measured according to the 301 F OECD protocol.

A measured volume of inoculated mineral medium, containing a known concentration of the test substance in order to reach about 50 to 100 mg ThOD/I (Theoretical Oxygen Demand) as the nominal sole source of organic carbon, was stirred in a closed flask (Oxitop^{™}respirometric flask) at a constant temperature (20 ± 2°C) for up to 28 days. Oxitop^{™} respirometric bottles were used in this test in order to access the biodegradability of the test samples: sealed culture BOD flasks were used at a temperature of 20±2 C during 28 days.

Evolved carbon dioxide was absorbed by pellets of Natrium or Potassium hydroxide present in the head space of the bottle. The amount of oxygen taken up by the microbial population (= oxygen consumption expressed in mg/l) during biodegradation process (biological oxidation of the test substance) decreased the pressure of the head space (Δ P measured by the pressure switch) and was mathematically converted in mg O₂ consumed /litre. Inoculum corresponded to a municipal activated sludge washed in mineral medium (ZW media) in order to decrease the DOC (Dissolved Oxygen Carbon) content. Control solutions containing the reference substance sodium acetate and also toxicity control (test substance + reference substance) were used for validation purposes. Reference substance, sodium acetate, has been tested in one bottle (at a nominal concentration of 129 mg/l corresponding to 100 mg ThOD/I) in order to check the viability of the inoculum. Toxicity control corresponds to the mixture of the substance reference and the test substance; it will check if the test substance is toxic towards the inoculum (if so, the test has to be redone at a lower test substance concentration, if feasible regarding the sensitivity of the method).

As the substances of the present invention are for a majority of them not very soluble in water (if some are soluble in water, their metabolite after hydrolysis containing the alkyl chain has often very low solubility in water), we used a specific protocol named the "emulsion protocol". This protocol enabled us to increase the bioavailability of the poorly water soluble substance in the aqueous phase where we had the inoculum.

Emulsion protocol consisted of adding the test substance in the bottle through a stock solution made in an emulsion.

Emulsion was a 50/50 v/v mixture of a stock solution of the test substance dissolved in a non-biodegradable surfactant (Synperonic^{®} PE 105 at 1 g/l) and then mixed with a mineral silicone oil AR 20 (Sigma).

The first dissolution of the test substance in the non-biodegradable surfactant solution often required magnetic stirrer agitation followed by ultrasonication.

Once the dissolution was made, we mixed the aqueous solution with a mineral silicone oil at a 50/50 volume/volume ratio. This emulsion was maintained by magnetic stirrer agitation and was sampled for an addition in the corresponding bottle in order to reach the required test substance concentration.

Two emulsion controls were run in parallel during the test in order to remove their value from the emulsion bottle containing the test substance added through the emulsion stock solution.

The result of the biodegradability test is provided in Table 2 here below:

**Table 2**

| | Biodegradability after 28 days |
|---|---|
| Compound of Example 1 | 92 % (OECD 301F) |

The result shows that the compound of example 1 has outstanding biodegradability. This beneficial effect is achieved without detrimentally affecting the surfactant properties of the ionic compounds.

Overall, the ionic compounds of the present invention show a good combination of surfactant properties combined with a good biodegradabilty, a combination which is in many cases not achieved by commercial surfactants.

Since the ionic compounds of the present invention are also easily available starting from internal ketones which are easily accessible from fatty acids or fatty acid derivatives, the ionic compounds of the present invention also provide economical benefits over the prior art compounds.

## Claims

1. A method for obtaining a quaternary ammonium compound having the general formula 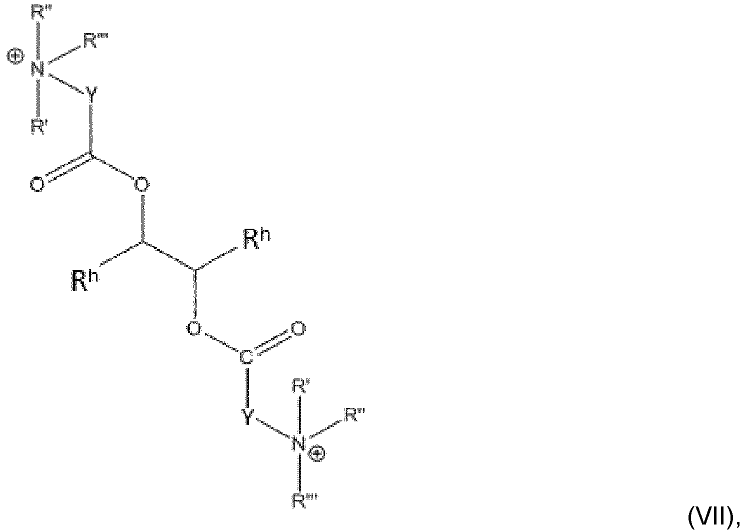 said method comprising reacting a diol of formula 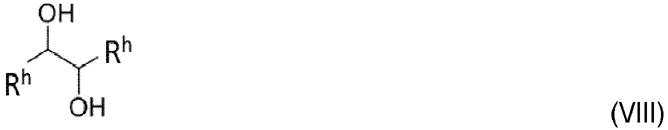 with an activated form of an amino-acid or derivative thereof or their hydrohalogenide adducts,
wherein
- the amino-acid or derivative thereof is of formula (IVa)
R'R"R'" ⁺N-Y-C(=O)-O⁻ (IVa),
and their hydrohalogenide adducts are of formula (IVb)
R'R"R'" ⁺N-Y-C(=O)-OH X⁻ (IVb),
- R^{h}, which may be the same or different at each occurrence, is an aliphatic group,
- R', R" and R'" are hydrogen or a C₁ to C₄ alkyl group,
- Y is a divalent C₁-C₆ aliphatic radical, and
- X denotes a halogen atom.

2. The method according to claim 1 wherein the activated form of the amino-acid or derivative thereof or their hydrohalogenide adducts is an acyl halogenide of formula (V)
R'R"R'" ⁺N-Y-C(=O)-X X⁻ (V)
wherein R', R", R"', Y and X are as defined in claim 1.

3. The method according to claim 2 which comprises obtaining the acyl halogenide of formula (V) by reacting the amino-acid or derivative thereof of formula (IVa) and/or a hydrohalogenide adduct thereof of formula (IVb) with a reagent chosen from a thionyl halogenide, an oxalyl halogenide, a phosphorus trihalogenide, a phosphorus pentahalogenide or a phosphorus oxyhalogenide.

4. The method according to claim 2 or 3 wherein X is chloride or bromide.

5. The method according to claim 4 wherein X is chloride.

6. The method according to claim 1 wherein the activated form of the amino-acid or derivative thereof or their hydrohalogenide adducts is an acyl azide of formula (VI)
R'R"R'" ⁺N-Y-C(=O)-N=N⁺=N⁻ (VI)
wherein R', R", R'" and Y are as defined in claim 1.

7. The method according to any one of claims 1 to 6 wherein R^{h} comprises at least 2, preferably at least 3 more preferably at least 4 carbon atoms.

8. The method according to claim 7 wherein R^{h} comprises from 10 to 20 carbon atoms.

9. The method according to claim 8 wherein R^{h} is a linear alkyl group.

10. The method according to any one of claims 1 to 9 wherein Y is chosen from -CH₂-, -CH(CH₃)-, -CH[CH(CH₃)₂]-, -CH[CH₂-CH(CH₃)₂]- and -CH[CH(CH₃)-CH₂-CH₃]-.

11. The method according to claim 10 wherein Y is -CH₂-.

12. The method according to any one of claims 1 to 11 wherein at least one of R', R" and R'" is a C₁ to C₄ alkyl group.

13. The method according to claim 12 wherein R' is a C₁ to C₄ alkyl group, R" is a C₁ to C₄ alkyl group and R'" is a C₁ to C₄ alkyl group.

14. The method according to claim 13 wherein R', R" and R'" are methyl.

15. The method according to any one of claims 1 to 14 wherein the amino-acid or derivative thereof of formula (IVa) is trimethyl glycine and the hydrohalogenide adduct of formula (IVb) is betaine hydrochloride.
